# EUROPEAN PATENT SPECIFICATION

(11) **EP 1 855 690 B1**
(45) Date of publication and mention of the grant of the patent: **01.06.2016**
(21) Application number: 06719046.2
(22) Date of filing: 20.01.2006
(51) Int. Cl.: A61K 31/65, A61K 9/16, A61P 31/04

(54) **A TETRACYCLINE METAL COMPLEX IN A SOLID DOSAGE FORM**
TETRACYCLIN-METALL-KOMPLEX IN EINER FESTEN DOSIERFORM
COMPLEXE METALLIQUE DE TETRACYCLINE SOUS FORME DE DOSAGE SOLIDE

(30) Priority: 21.01.2005 US 646357 P
(43) Date of publication of application: 21.11.2007
(73) Proprietor: Warner Chilcott Company, LLC, Puerto Rico 00738-1005 (PR)
(72) Inventor: DEVRIES, Tina, Long Valley, New Jersey 07853 (US); BOISSONNEAULT, Roger, Long Valley, New Jersey 07853 (US); MULDOON, Brendan, Glengormley, Newtownabbey, BT36 7DL (GB)
(74) Representative: O'Connell, Maura
(86) International application number: PCT/US2006/002070
(87) International publication number: WO 2006/078925

(56) References cited:
- EP-A- 0 096 942
- WO-A-2004/000223
- WO-A-2005/011707

## Description

### BACKGROUND OF THE INVENTION

### Field of the Invention

The present invention is directed to a method for making a dry granulation of a metal complex of the compound of Formula (I) in solid dosage form as claimed in Claim 1.

### Related Background Art

Tetracyclines are well, known antibiotic compounds that have been effectively used in the treatment of vibrio infections, gram-positive, gram-negative, aerobic and anaerobic bacteria, as well as spirochetes, mycoplasmas, rickettsiae, chlamydiae and some protozoans. Tetracyclines work by inhibiting enzyme reactions of bacterial cells, such as protein synthesis. It is commonly used in the treatment of bacterial infections caused by these organisms, such as urinary tract infections, upper respiratory tract infections, acne, gonorrhea, chlamydia, anthrax, lyme disease and others.

When administered orally, tetracyclines are absorbed in varying degrees. For example, 60-80% absorption is observed for demeclocycline, oxycycline, and tetracycline. While doxycycline and minocycline are absorbed at a higher level of about 90%.

It is widely held that tetracyclines should not be taken simultaneously with iron supplements, multivitamins, calcium supplements, antacids, or laxatives. Many believe that the efficacy and absorption of the tetracycline in the body decreases when taken simultaneously or in conjunction with one of the above noted products.

British Patent No. 1,360,998 to Villax describes a process for isolation of α-6-deoxytetracyclines from a crude reaction mixture. Villax also discloses that oral pharmaceutical suspensions may be prepared using the calcium salts of tetracyclines. The process taught by Villax uses methanol, i.e., an organic solvent. While the use of an organic solvent is not uncommon in preparing pharmaceutically active ingredients, it is generally preferred not to use them for preparing a solid dosage form. Therefore, the process disclosed in Villax for isolating deoxytetracycline salts would likely contain residual solvent, thus making this process unacceptable for producing a solid dosage form suitable for pharmaceutical administration.

Currently, only a suspension of a calcium salt of a tetracycline is available. Pharmaceutical suspensions, however, often suffer from a lack of patient compliance. In addition, many individuals do not prefer a liquid dosage form and find it inconvenient to use.

WO2004000223 discloses that mucositis is treated and/or prevented by administrating to a patient a rapidly disintegrating solid dosage form comprising a tetracycline. The tetracycline is preferably one that is poorly absorbed from the gastrointestinal tract. Such compositions have the advantage of treating the entire gastrointestinal tract since the active ingredient is not removed from the tract via absorption. Further, such compositions minimize systemic exposure and accompanying side effects. The compositions can be formulated as solid dosage forms comprising a tetracycline which disintegrates in an aqueous medium or saliva within in a short period, for example, two minutes. The dosage form can be, for example, a hard, compressed tablet adapted to rapidly disintegrate in saliva or an aqueous vehicle or a table prepared by freeze-drying a solution or suspension of the active ingredients.

However, making a solid dosage form of a tetracycline metal complex is extremely difficult. Processing hurdles exist and have not been adequately resolved. For example, a method of collecting the tetracycline metal complex from the aqueous suspension has not been achieved. Accordingly, it would be highly desirable to effectively produce a solid dosage form of a tetracycline metal complex.

### SUMMARY OF THE INVENTION

The present invention discloses a solid dosage form of a metal complex of a tetracycline of Formula (I) for pharmaceutical administration, 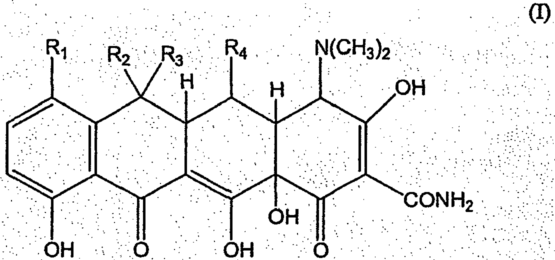 wherein
R₁ = Cl, N(CH₃)₂, or H;
R₂ = CH₃, H, or CH₂=;
R₃ = CH₃, H, OH, or absent; and
R₄ = OH or H,
with the proviso that if R₂ is CH₃ and R₃ is H, then R₄ is not OH.

A process for making a metal complex of the compound of Formula (I) in a solid dosage form is included in the present invention. The process comprises the steps of (i) providing an aqueous solution of the compound of Formula (I) or a physiologically acceptable salt thereof, where the compound is partially or completely solubilized; (ii) admixing a metal salt with the aqueous solution; (iii) admixing a suitable base to increase the pH of the aqueous solution, whereby a suspension of the metal complex of the compound of Formula (I) is formed after steps (ii) and (iii); and (iv) drying the suspension of the metal complex of the compound of Formula (I). The process includes the step of admixing one or more pharmaceutically acceptable excipients to form a granulation. The excipient added prior to the step of drying the suspension. A further step may involve filling the granulation into capsules or compressing the granulation into tablets.

The process for making a metal complex of the compound of Formula (I) in a solid dosage form comprises the steps of (i) providing an aqueous solution of Formula (I) or a physiologically acceptable salt thereof, where the compound is partially or completely solubilized; (ii) admixing a metal salt with the aqueous solution; (iii) admixing a suitable base to increase the pH of the aqueous solution, whereby a suspension of the metal complex of the compound of Formula (I) is formed after steps (ii) and (iii); (iv) admixing one or more pharmaceutically acceptable-excipients with the suspension, thereby forming a wet granulation; and (v) drying the wet granulation, thereby forming a dry granulation of the metal complex of the compound of Formula (I). The process may include, the step of admixing one or more pharmaceutically acceptable-excipients after the wet granulation is formed. In addition, the dry granulation may be filled into capsules or compressed into tablets.

The tetracycline metal complex of Formule (I) may be used in treating bacterial infections. The method of treatment comprises the step of administering an effective amount of a solid dosage form comprising a metal complex of a compound of Formula (I) for an effective period of time, to a host in need thereof.

In addition, the invention discloses a method for treating ailments resulting from microorganisms and/or bacteria, comprising the step of administering a safe and effective amount of a metal complex of a tetracycline of Formula (I) in a solid dosage form for an effective period of time, to a host in need thereof.

### DETAILED DESCRIPTION OF THE INVENTION

For the purposes of the present invention, all percentages provided are by weight, unless otherwise specified.

The tetracycline metal complex of Formula (I) and the active ingredients of the present invention are used in a "safe and effective amount." This is understood to mean a sufficient amount of a compound or composition that will positively modify the symptoms and/or condition to be treated, with the understanding that the amount is low enough to avoid serious side effects. The amount of the compound that is considered safe and effective will depend upon several factors. For example, the condition and severity of the condition being treated, the age, body weight, general health, sex, diet, and physical condition of the patient being treated, the duration of the treatment, the nature of concurrent therapy, the particular active ingredient being employed, the particular pharmaceutically-acceptable excipients utilized, the time of administration, method of administration, rate of excretion, drug combination, and any other relevant factors should be given consideration.

The term "pharmaceutically-acceptable excipient" is understood to mean any physiologically inert, pharmacologically inactive material known to one skilled in the art, which is compatible with the physical and chemical characteristics of the particular tetracycline metal complex of Formula (I) selected for use.

Tetracyclines represented by Formula (I)a are disclosed herein and can be combined with metal salts, such as calcium chloride, to produce metal complexes. Formula (I)a as disclosed herein is represented by the structure 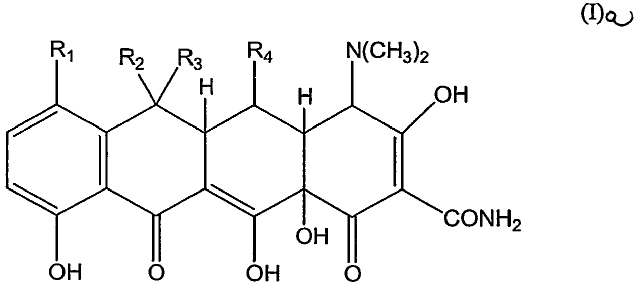 wherein
R₁ = Cl, N(CH₃)₂, or H;
R₂ = CH₃, H, or CH₂=;
R₃ = CH₃, H, OH, or absent; and
R₄ = OH or H,
with the proviso that if R₂ is CH₃ and R₃ is H, then R₄ is not OH.

An important aspect of the present invention is that the metal complex is formed in accordance to a molar ratio of tetracycline to metal ion ranging from about 3:1 to about 1:3. More preferably, the molar ratio is from about 2:1 to 1:1.

The present invention discloses a solid dosage form of a metal complex of a tetracycline of Formula (I) for pharmaceutically acceptable administration to a user/patient, i.e., any residual solvents or other impurities are at a level that is considered safe for human consumption. Formula (I) is minocycline where R₁ = N(CH₃)₂, and R₂, R₃ and R₄ = H.

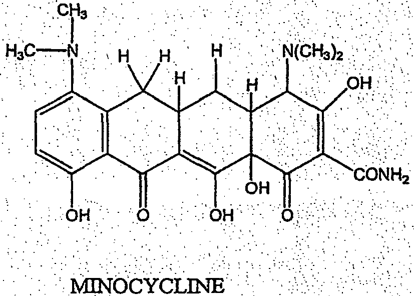

The metal complex of the compound of Formula (I) is selected from the group consisting of a calcium complex, a magnesium complex, a sodium complex, a zinc complex, an aluminum complex, an iron complex, a copper complex or mixtures thereof. Preferably, the metal complex is a calcium complex.

It is desirable that the tetracycline metal complex of the compound of Formula (I) undergo substantially complete complexation, where at least about 75% by weight of the tetracycline of Formula (I) has complexed. More preferably at least about 90% has complexed. It should be understood, however, that an excess of the tetracyclines of Formula (I) or metal salt may be added to form the metal complex of the compound of Formula (I).

Conventional wisdom dictates that the presence of a metal source with a tetracycline compound can interfere or inhibit the absorption of the tetracycline. Literature sources advise against the administration of the tetracycline with a calcium, iron, magnesium, or zinc supplement or product. Surprisingly, however, the inventors theorize that the body can effectively absorb a tetracycline from a metal complex of a tetracycline compound of Formula (I) in a solid dosage form.

Another aspect of the present invention is that the solid dosage form of the metal complex of the compound of Formula (I) can be engineered to exhibit a desired release profile. For example, the solid dosage form may be engineered to exhibit an immediate release, a controlled release, or a sustained release of the tetracycline compound of Formula (I). This may be accomplished, for example, by measuring the dissociation rates of the various metal complexes of the tetracycline compound of Formula (I) and then carefully selecting a particular complex based upon its properties. Moreover, two or more complexes may be combined to create the desired release profile.

The solid dosage form made by the process of the present invention may be a powder, capsule, tablet, coated tablet, aerosol, pellet, chewable tablet, lozenge, gelatin filled capsule, and the like.

The pharmaceutical dosage form made by the process of the present invention may be formulated together with one or more pharmaceutically acceptable carriers or excipients and/or bioactive agents.

Suitable pharmaceutically-acceptable excipients include, but are not limited to, polymers, resins, plasticizers, fillers, lubricants, binders, disintegrants, granulating agents, solvents, co-solvents, surfactants, preservatives, sweetening agents, flavoring agents, buffering systems, antioxidants, pharmaceutical grade dyes, pigments, and mixtures thereof.

Polymers that may be used, include but are not limited to, hydroxypropylmethylcellulose (HPMC) alone and/or in combination with hydroxypropylcellulose (HPC), carboxymethylcellulose, acrylic resins such as Eudragit®, methylcellulose, ethylcellulose, and polyvinylpyrrolidone or other commercially available film-coating preparations.

Suitable plasticizers, include but are not limited to, polyethylene glycol, propylene glycol, dibutyl phthalate, castor oil, acetylated monoglycerides, triacetin, and mixtures thereof.

Examples of fillers, include but are not limited to, lactose, sucrose, maltodextrin, mannitol, starch, microcrystalline cellulose, and mixtures thereof.

Lubricants that may be used, include but are not limited to, magnesium stearate, stearic acid, talc, and mixtures thereof.

Suitable binders, include but are not limited to, methycellulose, sodium carboxymethycellulose, liydroxypropylmethylcellulose, carbomer, povidone, acacia, guar gum, xanthan gum, tragacanth, calcium silicate, magnesium aluminum silicate, ethylcellulose, pregelatinized starch, and mixtures thereof. Particularly preferred are methycellulose, carbomer, xanthan gum, guar gum, povidone and sodium carboxymethycellulose.

Disintegrants that may be used, include but are not limited to, crospovidone, sodium carboxymethyl starch, sodium starch glycolate, sodium carboxymethyl cellulose, alginic acid, clays, ion exchange resins, and mixtures thereof.

Examples of surfactants, include but are not limited to, polyoxyethylene sorbitan fatty acid esters, polyoxyethylene monoalkyl ethers, sucrose monoesters, lanolin esters and ethers, and mixtures thereof.

Suitable preservatives, include but are not limited to, phenol, alkyl esters of parahydroxybenzoic acid, benzoic acid and the salts thereof, boric acid and the salts thereof, sorbic acid and the salts thereof, chlorbutanol, benzyl alcohol, thimerosal, phenylmercuric acetate and nitrate, nitromersol, benzalkonium chloride, cetylpyridinium chloride, methyl paraben, propyl paraben, and mixtures thereof. Particularly preferred are the salts of benzoic acid, cetylpyridinium chloride, methyl paraben and propyl paraben.

Antioxidants that may be used, include but are not limited to, tocopherols and derivatives thereof, ascorbic acid, beta-carotene, selenium, sodium bisulfite, sodium metabisulfite, ascorbyl palmitate, citric acid, catechins and derivatives thereof, and mixtures thereof.

Suitable sweeteners, include but are not limited to, sucrose, glucose, saccharin, aspartame, sorbitol, xylitol, sucralose and mixtures thereof. Particularly preferred are sucrose and sucralose.

Buffering systems that may be used include, but are not limited to, potassium acetate, boric carbonic, phosphoric, succinic, malic, tartaric, citric, acetic, benzoic, lactic, glyceric, gluconic, glutaric, glutamic, and mixtures thereof. Particularly preferred are phosphoric, tartaric, citric, and potassium acetate.

A variety of solvents may be used. However, water is the preferred solvent. The water may be acidified with an acidifying agent. Inorganic acidifying agents include, but are not limited to, hydrochloric acid, sulfuric acid, nitric acid, and mixtures thereof. Alternatively, organic acidifying agents may be used, such as for example, acetic acid, ethanoic acid, and mixtures thereof. Preferably the acidifying agent is hydrochloric acid.

Suitable co-solvents, include but are not limited to, ethanol, glycerin, propylene glycol, polyethylene glycol, and mixtures thereof.

The pharmaceutical compositions described herein are comprised of from about 0.1 weight percent (wt.%) to about 99.9 wt.%, preferably from about 5.0 wt.% to about 50.0 wt.%, and most preferably from about 10 wt.% to about 50 wt.% of the tetracycline metal complex of Formula (I), and from about 0.1 wt.% to about 99.9 wt.%, preferably from about 5.0 wt.% to about 99.9 wt.%, and most preferably from about 50 wt.% to about 90 wt.% of one or more pharmaceutically-acceptable excipients.

The solid dosage form of the metal complex of the compound of Formula (I) is made using a process comprising the steps of (i) providing an aqueous solution of a tetracycline of Formula (I) or a physiologically acceptable salt thereof, where the compound is partially or completely solubilized; (ii) admixing a metal salt to the aqueous solution, (iii) admixing a base to increase the pH of the aqueous solution, whereby a suspension of a tetracycline metal complex of Formula (I) is formed after steps (ii) and (iii); and (iv) drying the suspension of the tetracycline metal complex of Formula (I). One or more pharmaceutically acceptable-excipients is admixed, before the drying step.

Examples of the physiologically acceptable salts, include, but are not limited to, hyclates, monohydrates, carrageenates, hydrochlorides or phosphates of the tetracycline of Formula (I). The tetracycline of Formula (I) or physiologically acceptable salts thereof are partially or completely dissolved in an aqueous solution. Any suitable means may be used to dissolve the tetracycline of Formula (I) or its salt in the aqueous solution. Generally, all that is required is some form of mixing. Other components, such as ethanol or acid, may be included in the aqueous solution. The aqueous solution generally will have a pH that is in a range of about 0.5 to about 8.

Suitable metal salts include but are not limited to, calcium salts, sodium salts, magnesium salts, zinc salts, and mixtures thereof. A particularly preferred metal salt is calcium chloride. The metal salt may be added with or without mixing, but is generally mixed into the aqueous solution.

About 1 wt.% to about 70 wt.%, preferably about 10 wt.% to about 50 wt.%, and more preferably about 35 wt.% to about 50 wt.% of the metal salt solution is added to the aqueous solution, whereby the ratio of metal to tetracycline is from about 10:1 to about 0.5:1, such that the resulting complex has a molar ratio of tetracycline to metal ion ranging from about 3:1 to about 1:3.

A pharmaceutically acceptable base is added to the aqueous solution and mixed with the other components. Examples of pharmaceutically acceptable bases capable of forming salts for the purpose of the present invention, include, but are not limited to, alkali metal bases, such as sodium hydroxide, potassium hydroxide, lithium hydroxide and the like; alkaline earth metal bases, such as calcium hydroxide, barium hydroxide and the like; and ammonium hydroxide. Alkali metal or alkaline earth metal salts suitable for forming pharmaceutically acceptable salts can include anions such as carbonates, bicarbonates such as sodium bicarbonate and sulfates. Additional suitable bases include for example, triethanolamine, diethanolamine, monoethanolamine, triethylamine and mixtures thereof.

The base is added in an amount effective to form a suspension of the metal complex of the compound of Formula (I). Generally the addition of the base raises the pH of the solution to a pH range of about 2 to about 14, preferably about 5 to about 10, and most preferably about 5 to about 7. Several factors are used to determine the amount of base that will be added, including the pH of the aqueous solution, the pKa(s) of the tetracycline, and the equilibrium constant of the tetracycline metal complex.

The suspension of the metal complex of the compound of Formula (I) is formed after the addition of the metal salt and the suitable base. It should be understood, however, that the order of addition of these ingredients may be varied. In a preferred embodiment, the metal salt is added and mixed into the aqueous solution prior to the addition of the suitable base.

Drying the suspension may be carried out using a variety of techniques. For example in one embodiment, a spray dryer is used and the suspension is sprayed onto an excipient. Other methods include decanting, evaporation, freeze drying, tray drying, fluid-bed drying, and the like.

The process for making a solid dosage form of a metal complex of the compound of Formula (I) comprises the steps of: (i) providing an aqueous solution of the compound of Formula (I) or a physiologically acceptable salt thereof, where the compound is partially or completely solubilized; (ii) admixing a metal salt with the aqueous solution, (iii) admixing a base to increase the pH of the solution, whereby a suspension of the metal complex with the compound of Formula (I) is formed after steps (ii) and (iii); (iv) admixing one or more pharmaceutically acceptable-excipients with the suspension, thereby forming a wet granulation; and (v) drying the wet granulation, thereby forming a dry granulation of the metal complex of the compound of Formula (I). Steps (i)-(iii) of this embodiment are the same as the prior described process that forms the solid dosage form from the suspension.

This embodiment, however, utilizes an excipient to assist in forming a wet granulation. The inventors have discovered that an excipient, e.g., microcrystalline cellulose, can be used to absorb and adsorb the moisture in the suspension when admixed with the suspension. The result is a wet granulation or slurry that has about 5 wt.% to about 99 wt.% water. Preferably, the moisture in the wet granulation is about 25 wt.% to about 60 wt.%.

One or more pharmaceutically acceptable-excipients can be admixed in either process during any of the process steps. Moreover, excipients can be added in more than one step.

The wet granulation may be dried by tray-drying, fluid-bed drying, decanting, evapoiating, freeze drying, a combination thereof, or by other processes known to those skilled in the art.

To ensure that dire solid dosage form is suitably stable, it is desirable that the granulation of the metal complex of the compound of Formula (I) be dried to a moisture content of about 1 wt.% to about 15 wt.% based on the total weight of the metal complex granulation. More preferably, the moisture content should be less than about 10 wt.%. Most preferably, to about 1 wt.% to about 6 wt.%.

The resulting metal complex of the compound of Formula (I) is in the form of a dry granulation consisting of granules and powder. The dry granulation may be blended with excipients such as lubricants, e.g., magnesium stearate. The final blend may be further processed, for example, by filling it into capsules.

The solid dosage form is generally administrated orally. Suitable forms include, but are not limited to, tablets, capsules, powders, granules, lozenges, aerosols, pellets, chewable tablets, and the like.

The inventors have also discovered that the granulation formed after the drying step is well suited for tableting. In fact, tablets can be made directly from the metal complex suspension, making the process more efficient. However, it should be understood that tableting excipients may be incorporated. As previously noted, the excipients, such as lubricants, may be added before and/or after the step of drying the suspension or wet granulation. The excipients are simply added and mixed with the other components.

In a preferred embodiment, the tablets are chewable tablets. Moreover, the inventors have discovered that the insoluble nature of the metal complex helps mask the unpleasant taste of the tetracycline compound of Formula (I). Typically, these compounds are known to have an extremely bitter taste.

In addition, the solid dosage form made by the process of the present invention may contain additional ingredients. For example, the additional ingredients may include natural and artificial flavors, sweeteners, colorings, coating excipients, binders, disintegrants, lubricants, and the like.

Excipents such as binding agents, for example syrup, acacia, gelatin, sorbitol, tragacanth, or polyvinyl-pyrrolidone; fillers for example lactose, sugar, maize-starch, calcium phosphate, sorbitol or glycine; tableting lubricant, for example magnesium stearate, talc, polyethylene glycol or silica; disintegrants for example potato starch, or acceptable wetting agents such as sodium lauryl sulphate, may be included to facilitate the formation of the tablet. In addition, coatings may be applied over the tablets using methods known in the art.

Bacterial infections may be treated using the solid dosage form disclosed herein. The method of treatment comprises the step of administering to a host, such as a human or animal, a safe and effective amount of a tetracycline metal complex, in solid dosage form. Examples of bacterial infections that can be treated with a tetracycline metal complex, include urinary tract infections, upper respiratory tract infections, acne, gonorrhea, chlamydia, syphilis, anthrax, lyme disease and the like.

The solid dosage form disclosed herein may also be used in the treatment of ailments caused by bacteria and microorganisms. Non-limiting examples of bacteria and microorganisms are gram-positive microorganisms, gram-negative microorganisms, aerobic bacteria, anaerobic bacteria, spirochetes, mycoplasmas, rickettsiae, chlamydiae, treponema, listeria, bacillus anthracis, fusobacterium fusiforme, actinomyces israelii, clostridium, ureaplasma urealyticum, borrelia recurrentis, haemophilus ducreyi, yersinia pestis, francisella tularensis, vibrio cholerae, brucella, campylobacter fetus, bartonella bacilliformis, calymmatobacterium granulomatis, and protozoans. The method of treatment comprises require administering a safe and effective amount of a solid dosage form of a tetracycline metal complex for an effective period of time.

### EXAMPLE 1

83 grams of minocycline hydrochloride was added to 248 grams of water. Hydrochloric acid was then added to adjust the pH to less than 1. Next, 37.3 grams of 40% w/w calcium chloride solution was added. The solution was then mixed. This step was followed by adding 130 grams of 5N sodium hydroxide solution. Addition of the base, formed and precipitated a minocycline calcium complex suspension, which had a final pH in the range of from about 5 to less than about 8.

### EXAMPLE 2

600 grams of microcrystalline cellulose is added to a suspension of minocycline calcium complex made following the procedure of Example 1. The suspension is mixed to form a wet granulation. The wet granulation is subjected to a drying operation using a tray dryer until the moisture content is less than about 5% w/w. The dried granulation is then passed through a mill, to reduce the particle size of the granulation to a particle size suitable for making into a solid dosage form. Next, 2 grams of magnesium stearate is added and blended into the mixture. Finally, the mixture is compressed into tablets containing 75 mg of minocycline. The tablets may then be coated with a film coating.

It should be understood that different levels of minocycline may be delivered in tablet form.

### EXAMPLE 3

The procedure of Example 1 is followed to form a minocycline calcium complex. 500 grams of microcrystalline cellulose is then mixed into the minocycline calcium complex that is formed, resulting in a wet granulation. The wet granulation is dried in a tray dryer until the moisture content is less than about 5 wt.%. The dried granulation is then passed through a mill, to reduce the particle of the granulation to a particles size suitable for making into a solid dosage form. Next, 2 grams of magnesium stearate is added and blended into the mixture. The dry granulation is then filled into size 00 hard gelatin capsules, where each capsule contains 50 mg of minocycline.

It should be understood that different levels of minocycline may be delivered in capsule form.

### EXAMPLE 4

83 grams of minocycline hydrochloride was added to 248 grams of purified water. Hydrochloric acid (37% w/w) was then added to lower the pH to less than 1. Next 20 g of calcium chloride dihydrate was added and mixed. This step was followed by adding and mixing 201 grams of 20% w/w sodium hydroxide solution. Addition of the base formed and precipitated a minocycline calcium complex suspension. The suspension that was formed had a pH reading of 12. Next, 42 grams of HCl (37% w/w) was added and mixed. The final pH of the suspension was 7.

### EXAMPLE 5

25 g of croscarmellose sodium and 175 g microcrystalline cellulose are added to a suspension of minocycline calcium complex made following the procedure of EXAMPLE 1 or EXAMPLE 4. The suspension is mixed to form a wet granulation. The wet granulation is subjected to a drying operation using a fluid bed dryer until the moisture content is less than about 5% w/w. The dried granulation is then passed through a mill, to reduce the particle of the granulation to a particles size suitable for making into a solid dosage form. Next 150 grams of microcrystalline cellulose is added and blended into the mixture. This is followed by adding and blending 2 grams magnesium stearate into the mixture. Finally, the mixture is compressed into tablets containing 100 mg of minocycline. The tablets may then be coated with a film coating.

It should be understood that different levels of minocycline may be delivered in tablet form.

### EXAMPLE 6

15 grams of croscarmellose sodium, 30 grams of starch, 4.5 grams of citric acid and 150 grams of microcrystalline cellulose are added to a suspension of minocycline calcium complex made following the procedure of EXAMPLE 1 or EXAMPLE 4. The suspension is mixed to form a wet granulation. The wet granulation is subjected to a drying operation using a fluid bed dryer until the moisture content is less than about 5% w/w. The dried granulation is then passed through a mill, to reduce the particle of the granulation to a particles size suitable for making into a solid dosage form. Next 150 grams of microcrystalline cellulose is added and blended into the mixture. This is followed by adding and blending 2 grams of magnesium stearate into the mixture. Finally, the mixture is compressed into tablets containing 100 mg of minocycline. The tablets may then be coated with a film coating.

It should be understood that different levels of minocycline may be delivered in tablet form.

## Claims

1. A process for making a dry granulation of a metal complex of a compound of Formula (I) in a solid dosage form, 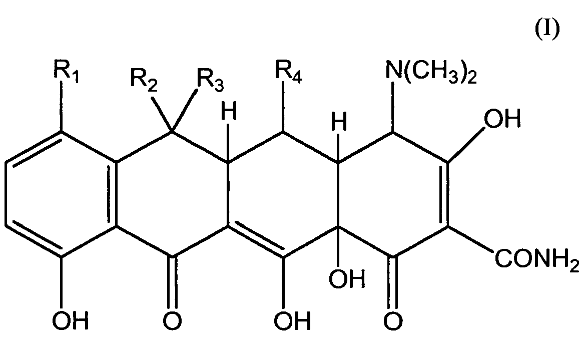 wherein
R₁ = N(CH₃)₂;
R₂ = H;
R₃ = H; and
R₄ = H,
wherein the compound is minocycline and wherein said metal complex is selected from the group consisting of a calcium complex, a magnesium complex, a sodium complex, a zinc complex, an aluminum complex, an iron complex, a copper complex and mixtures thereof, said process comprising the steps of:
(i) providing an aqueous solution of the compound of Formula (I) or a physiologically acceptable salt thereof, where the compound is partially or completely solubilized;
(ii) admixing a metal salt with said aqueous solution,
(iii) admixing a base to increase the pH of said aqueous solution, whereby a suspension of a metal complex of the compound of Formula (I) is formed after steps (ii) and (iii); and
(iv) admixing one or more pharmaceutically acceptable-excipients with the suspension thereby forming a wet granulation; and
(v) drying the wet granulation, thereby forming a dry granulation of the metal complex of the compound of Formula (I).

2. The process of claim 1, wherein said suspension of the metal complex of the compound of Formula (I) provided in step (iii) has a pH range of 2 to 14.

3. The process of claim 1, wherein said metal salt is selected from the group consisting of calcium salts, magnesium salts, sodium salts, zinc salts, and mixtures thereof.

4. The process of claim 1, wherein said metal salt is calcium chloride.

5. The process of claim 1, wherein said base is selected from the group consisting of sodium hydroxide, triethanolamine, and mixtures thereof.

6. The process of claim 1, wherein said granulation formed in step (v) has a moisture level of less than 10 wt.%.

7. The process of claim 1, further comprising the step of:
(a) compressing said dry granulation, thereby forming tablets; or
(b) filling gelatin capsules with said dry granulation.

8. The process of claim 1, wherein the physiologically acceptable salt of the compound of Formula (I) is selected from the group consisting of a hyclate, monohydrate, carrageenate, hydrochloride, phosphate, and mixtures thereof.

9. The process of claim 1, wherein the pharmaceutically acceptable-excipient in step (iv) is microcrystalline cellulose.

10. A process for making a pharmaceutical solid dosage form, comprising making the dry granulation of the metal complex of the compound of Formula (I) in accordance with the process of claim 1 and either filling the granulation into capsules or compressing the granulation into tablets.

11. The process of claim 1 or 10, wherein said metal complex is a calcium complex.

12. The process of claim 1 or 10, wherein the compound of the complex of Formula (I) has a release profile that is immediate, controlled, or sustained.

13. The process of claim 1, wherein said solid dosage form is a pharmaceutical dosage form selected from the group consisting of powders, granules, tablets, coated tablets, gelatin filled capsules, pellets, and chewable tablets.

14. The process of claim 13, wherein said solid dosage form is a chewable tablet.

15. The process of claim 7, wherein the tablets are chewable tablets.

## Patentansprüche

1. Verfahren für die Herstellung einer trockenen Granulierung eines Metallkomplexes einer Verbindung der Formel (I) in einer festen Dosierungsform, 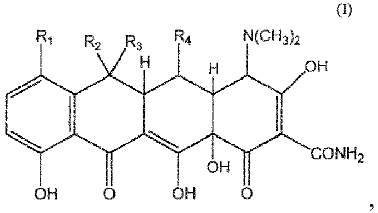 wobei
R₁ = N(CH₃)₂;
R₂ = H;
R₃ = H; und
R₄ = H,
wobei die Verbindung Minocyclin ist und wobei der Metallkomplex aus der Gruppe ausgewählt ist, die aus Folgenden besteht: einem Calciumkomplex, einem Magnesiumkomplex, einem Natriumkomplex, einem Zinkkomplex, einem Aluminiumkomplex, einem Eisenkomplex, einem Kupferkomplex und Mischungen davon, wobei das Verfahren die folgenden Schritte umfasst:
(i) Bereitstellen einer wässrigen Lösung der Verbindung der Formel (I) oder eines physiologisch verträglichen Salzes davon, wo die Verbindung teilweise oder vollständig solubilisiert ist;
(ii) Einmischen eines Metallsalzes in die wässrige Lösung;
(iii) Einmischen einer Base, um den pH der wässrigen Lösung zu erhöhen, wodurch eine Suspension eines Metallkomplexes der Verbindung der Formel (I) nach den Schritten (ii) und (iii) gebildet wird; und
(iv) Einmischen von einem oder mehreren pharmazeutisch verträglichen Hilfsstoffen in die Suspension, wodurch eine nasse Granulierung gebildet wird; und
(v) Trocknen der nassen Granulierung, wodurch eine trockene Granulierung des Metallkomplexes der Verbindung der Formel (I) gebildet wird.

2. Verfahren nach Anspruch 1, wobei die Suspension des Metallkomplexes der Verbindung der Formel (I), die im Schritt (iii) bereitgestellt wurde, einen pH-Bereich von 2 bis 14 aufweist.

3. Verfahren nach Anspruch 1, wobei das Metallsalz aus der Gruppe ausgewählt ist, die aus Folgenden besteht: Calciumsalzen, Magnesiumsalzen, Natriumsalzen, Zinksalzen und Mischungen davon,

4. Verfahren nach Anspruch 1, wobei das Metallsalz Calciumchlorid ist.

5. Verfahren nach Anspruch 1, wobei die Base aus der Gruppe ausgewählt ist, die aus Natriumhydroxid, Triethanolamin und Mischungen davon besteht.

6. Verfahren nach Anspruch 1, wobei die Granulierung, die im Schritt (v) gebildet wurde, einen Feuchtigkeitsgehalt von weniger als 10 Gew.-% aufweist.

7. Verfahren nach Anspruch 1, das weiter den folgenden Schritt umfasst:
(a) Verpressen der trockenen Granulierung, wodurch Tabletten gebildet werden; oder
(b) Füllen von Gelatinekapseln mit der trockenen Granulierung.

8. Verfahren nach Anspruch 1, wobei das physiologisch verträgliche Salz der Verbindung der Formel (I) aus der Gruppe ausgewählt ist, die aus Folgenden besteht: einem Hyclat, Monohydrat, Carrageenat, Hydrochlorid, Phosphat und Mischungen davon.

9. Verfahren nach Anspruch 1, wobei der pharmazeutisch verträgliche Hilsstoff im Schritt (iv) mikrokristalline Cellulose ist.

10. Verfahren zur Herstellung einer pharmazeutischen festen Dosierungsform, das das Herstellen der trockenen Granulierung des Metallkomplexes der Verbindung der Formel (I) gemäß dem Verfahren nach Anspruch 1 und entweder das Füllen der Granulierung in Kapseln oder das Verpressen der Granulierung zu Tabletten umfasst.

11. Verfahren nach Anspruch 1 oder 10, wobei der Metallkomplex ein Calciumkomplex ist.

12. Verfahren nach Anspruch 1 oder 10, wobei die Verbindung des Komplexes der Formel (I) ein Profil der Freisetzung aufweist, die sofort, kontrolliert oder verzögert erfolgt.

13. Verfahren nach Anspruch 1, wobei die feste Dosierungsform eine pharmazeutische Dosierungsform ist, die aus der Gruppe ausgewählt ist, die aus Folgenden besteht: Pulvern, Granulaten, Tabletten, überzogenen Tabletten, gefüllten Gelatinekapseln, Pellets und Kautabletten.

14. Verfahren nach Anspruch 13, wobei die feste Dosierungsform eine Kautablette ist.

15. Verfahren nach Anspruch 7, wobei die Tabletten Kautabletten sind.

## Revendications

1. Procédé de préparation d'une granulation sèche d'un complexe métallique d'un composé de Formule (I) sous une forme de dosage solide, 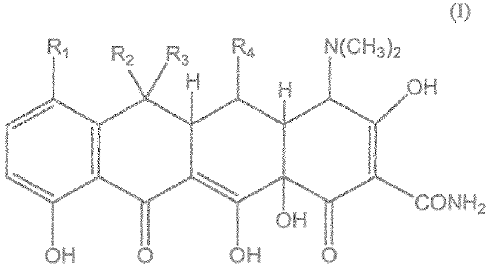 où
R₁ = N(CH₃)₂;
R₂ = H;
R₃ = H ; et
R₄ = H ;
dans lequel le composé est la minocycline et où ledit complexe métallique est choisi dans le groupe constitué par un complexe de calcium, un complexe de magnésium, un complexe de sodium, un complexe de zinc, un complexe d'aluminium, un complexe de fer, un complexe de cuivre et des mélanges de ceux-ci, ledit procédé comprenant les étapes consistant à :
(i) fournir une solution aqueuse du composé de Formule (I) ou d'un sel physiologiquement acceptable de celui-ci, où le composé est partiellement ou complètement solubilisé ;
(ii) mélanger un sel métallique avec ladite solution aqueuse ;
(iii) mélanger une base afin d'augmenter le pH de ladite solution aqueuse, ce par quoi une suspension d'un complexe métallique du composé de Formule (I) est formée après les étapes (ii) et (iii) ; et
(iv) mélanger un ou plusieurs excipients pharmaceutiquement acceptables avec la suspension, formant ainsi une granulation humide ; et
(v) sécher la granulation humide, formant ainsi une granulation sèche du complexe métallique du composé de Formule (I).

2. Procédé selon la revendication 1, dans lequel ladite suspension du complexe métallique du composé de Formule (I) fournie dans l'étape (iii) possède une plage de pH allant de 2 à 14.

3. Procédé selon la revendication 1, dans lequel ledit sel métallique est choisi dans le groupe constitué par les sels de calcium, les sels de magnésium, les sels de sodium, les sels de zinc, et des mélanges de ceux-ci.

4. Procédé selon la revendication 1, dans lequel ledit sel métallique est le chlorure de calcium.

5. Procédé selon la revendication 1, dans lequel ladite base est choisie dans le groupe constitué par l'hydroxyde de sodium, la triéthanolamine, et des mélanges de ceux-ci.

6. Procédé selon la revendication 1, dans lequel ladite granulation formée dans l'étape (v) possède un taux d'humidité inférieur à 10% en poids.

7. Procédé selon la revendication 1, comprenant en outre l'étape consistant à :
(a) comprimer ladite granulation sèche, formant ainsi des comprimés ; ou
(b) remplir des capsules de gélatine avec ladite granulation sèche.

8. Procédé selon la revendication 1, dans lequel le sel physiologiquement acceptable du composé de Formule (I) est choisi dans le groupe constitué par un hyclate, un monohydrate, un carraghénate, un chlorhydrate, un phosphate, et des mélanges de ceux-ci.

9. Procédé selon la revendication 1, dans lequel l'excipient pharmaceutiquement acceptable dans l'étape (iv) est la cellulose microcristalline.

10. Procédé de préparation d'une forme de dosage solide pharmaceutique, comprenant la préparation de la granulation sèche du complexe métallique du composé de Formule (I) conformément au procédé selon la revendication 1, et charger la granulation dans des capsules ou bien mettre la granulation sous forme de comprimés.

11. Procédé selon la revendication 1 ou 10, dans lequel ledit complexe métallique est un complexe de calcium.

12. Procédé selon la revendication 1 ou 10, dans lequel le composé du complexe de Formule (I) possède un profil de libération qui est immédiat, contrôlé ou prolongé.

13. Procédé selon la revendication 1, dans lequel ladite forme de dosage solide est une forme de dosage pharmaceutique choisie dans le groupe constitué par les poudres, les granulés, les comprimés, les comprimés enrobés, les capsules de gélatine remplies, les pastilles et les comprimés croquables.

14. Procédé selon la revendication 13, dans lequel ladite forme de dosage solide est un comprimé croquable.

15. Procédé selon la revendication 7, dans lequel les comprimés sont des comprimés croquables.
